# EUROPEAN PATENT APPLICATION

(11) **EP 3 087 978 A1**
(43) Date of publication of application: **02.11.2016**
(21) Application number: 14874183.8
(22) Date of filing: 19.12.2014
(51) Int. Cl.: A61K 9/20, A61K 9/24, A61K 9/28, A61K 47/38

(54) **SUSTAINED-RELEASE PHARMACEUTICAL COMPOSITION CONTAINING ACEBROPHYLLINE AND HYDROPHILLIC SUSTAINED-RELEASE AGENT**

(30) Priority: 26.12.2013 KR 20130164680
(71) Applicant: Hyundai Pharm Co., Ltd., Cheonan-si, Chungcheongnam-do 330-911 (KR)
(72) Inventor: LEE, Pung Sok, Yongin-si Gyeonggi-do 446-718 (KR); LEE, Chung Lyol, Icheon-si Gyeonggi-do 467-822 (KR); KIM, Seong Yeon, Goyang-si Gyeonggi-do 412-220 (KR); NAM, Yun Won, Hwaseong-si Gyeonggi-do 445-897 (KR)
(74) Representative: Scholz, Volker
(86) International application number: PCT/KR2014/012603
(87) International publication number: WO 2015/099365

(57) **Abstract**

The present invention relates to a pharmaceutical composition containing acebrophylline thereby having both immediate and extended release characteristics, and more specifically, to a sustained-release pharmaceutical composition containing acebrophylline and a hydrophillic sustained-release agent having a viscosity of no more than 13,000 cps.

## Description

### Technical Field

The present invention relates to a pharmaceutical composition containing acebrophylline and having both a fast-acting property and a long-acting property. The present invention relates to a sustained-release pharmaceutical composition containing acebrophylline, and a hydrophilic sustained-release agent having a viscosity of 13,000 cps or less.

### Background Art

Acebrophylline is a compound synthesized by salifyingambroxol with 7-theophylline and a salt composed of an acid and a base. Acebrophylline is metabolized by being separated into ambroxol and 7-theophylline when orally administered. Acebrophylline is selectively applied to the bronchial or lung tissue to inhibit the activity of phospholipase in bronchial alveolus, and to improve the alveolar surface activity, thereby exhibiting the sputum-removal action. In addition, acebrophylline suppresses the production of leukotrienes (LTs) and prostaglandins (PGs) to exhibit a strong anti-inflammatory action, and thus recovers or expands the convulsed bronchial tubes to a normal state by deteriorating bronchial hypersensitiveness.

Acebrophylline preparations previously licensed in Korea, (for example, product name Surfolase® capsule(Hyundai Pharmaceutical), of which 100 mg is administered twice daily, as compared with a drug administered once daily or a drug administered thrice daily (before and after meals)), have unclear administration time points, causing a reduction in patient drug compliance and an irregular administration time (interval), resulting in undesired therapeutic effects. In order to improve the drug compliance, it is preferable to develop sustained-release agents that can exhibit a continuous pharmaceutical effect. However, such sustained-release agents exhibit only continuous pharmaceutical effects without fast expression of pharmaceutical effects, and thus cannot be expected to have fast administration effects in, for example, respiratory diseases that are in need of fast expression of pharmaceutical effects, so the sustained-release agents increase the inconvenience of patients and are not expected to produce desired therapy effects as compared with agents on the market. Accordingly, in order to complement these defects, acebrophylline agents need to be developed that have both a fast-acting property to rapidly reach the effective blood concentration after administration to exhibit pharmaceutical effects, and also have a long-acting property to sustain pharmaceutical effects for a long period of time.

Thus, the present inventors studied to develop acebrophylline preparations that have both immediate-release characteristics of rapidly reaching the effective blood concentration after administration to exhibit drug efficacy, and sustained-release characteristics of sustaining drug efficacy for a long time. As a result, the present inventors developed an acebrophylline preparation that, while exhibiting fast drug efficacy and sustaining therapeutic effects, has an improved convenience of administration by pharmaceutically modifying the acebrophylline administration schedule from 100 mg twice daily into 200 mg once daily, and thus completed the present invention.

### Detailed Description of the Invention

### Technical Problem

An aspect of the present invention is to provide a pharmaceutical composition containing acebrophylline and simultaneously having a fast-acting property, a long-acting property, and the convenience of administration, simultaneously.

Another aspect of the present invention is to provide a method for imparting, to acebrophylline, initial immediate-release characteristics and late sustained-release characteristics.

### Technical Solution

In accordance with an aspect of the present invention, there is provided a sustained-release pharmaceutical composition containing acebrophylline and a hydrophilic sustained-release agent having a viscosity of 13,000 cps or less.

Hereinafter, the present invention will be described in detail.

The present inventors carried out experiments to prepare a pharmaceutical composition containing acebrophylline, and as a result, the present invention verified that the use of a hydrophilic sustained-release agent having a viscosity of 13,000 cps or less resulted in an excellent initial dissolution rate (test example 1), thereby expecting an increase in the convenience of administration and an improvement in therapeutic effect, and thus completed the present invention.

The pharmaceutical composition of the present invention contains acebrophylline as an active ingredient, and exhibits a fast dissolution rate although it contains the hydrophilic sustained-release agent, and thus has both a fast-acting property and a long-acting property.

Here, acebrophylline is a compound (ambroxol[trans-4-2-amino-3,5-dibromobenzy]amino cyclohexanol with theophylline-7-acetic acid[1,3-dimethylxanthine-7-acetic acid]) that is synthesized by salifyingambroxol with 7-theophylline and a salt composed of an acid and a base.

Acebrophylline is metabolized by being separated into ambroxol and 7-theophylline when orally administered. Acebrophylline is selectively applied to the lung tissue to exhibit the sputum-removal action, and inhibits the production of leukotrienes and prostaglandins to exhibit an anti-inflammatory action. Therefore, acebrophylline can be used in the prevention or treatment of acute respiratory disease, chronic respiratory disease, acute bronchitis, chronic bronchitis, bronchial asthma, sinusitis, or rhinitis sicca.

Therefore, preferably, the present invention achieves fast drug efficacy exhibition and continuous drug efficacy retention of acebrophylline, and may be used in the prevention or treatment of at least one disease selected from the group consisting of acute respiratory disease, chronic respiratory disease, acute bronchitis, chronic bronchitis, bronchial asthma, sinusitis, and rhinitis sicca.

As a preferable example, the content of acebrophylline contained in the composition of the present invention may be easily determined by a person skilled in the art depending on the age, sex, disease severity, weight, and the like of a patient, but may be, preferably, a total of 20-250 mg, and more preferably, a total of 200 mg. Less than 20 mg of acebrophylline may not sufficiently exhibit a desired drug efficacy, and more than 250 mg of acebrophylline may cause side effects due to an excessive pharmacological action and may spoil administration due to an increase in the weight of the composition *per se*, and thus is not useful as a pharmaceutical composition.

Here, the hydrophilic sustained-release agent refers to a hydrophilic material that is used to release drugs for a long time. The term "hydrophilic" refers to having a feature of strongly interacting with water to be dissolved in water with strong affinity with water. Broadly, highly polar or ionic materials or atomic groups exhibit hydrophilicity.

The hydrophilic sustained-release agent has a viscosity of 13,000 cps or less, and specifically, 100-13,000 cps, 500-13,000 cps, 800-13,000 cps, 1000-13,000 cps, 100-11,000 cps, 500-11,000 cps, 800-11,000 cps, or 1000-11,000 cps. The use of a hydrophilic sustained-release agent having a viscosity under the above viscosity ranges fails to the formation of a matrix, or even if formed, the matrix is weakly formed, and thus the initial drug release bursts, causing side effects. While, the use of the hydrophilic sustained-release agent having a viscosity exceeding the viscosity ranges results in the formation of excessive matrixes, thereby failing to the drug release (trapping), and thus, an expected effect cannot be exhibited.

The hydrophilic sustained-release agent is at least one selected from the group consisting of, but not limited to, for example, agar, carbomer, alginic acid, sodium alginate, carrageenan, guar gum, acacia, hypromellose (HPMC), hypromellose acetate succinate, hypromellose phthalate, methylcellulose, polyethylene oxide, polyvinyl alcohol, sodium hyaluronate, xanthan gum, polycarbophil, povidone, ceratonia, chitosan, and a mixture thereof.

In addition, the hydrophilic sustained-release agent may be contained in less than 20 wt% on the basis of the entire composition, but is not limited thereto. Preferably, the hydrophilic sustained-release agent may be contained in 3-15 wt%. The sustained-release agent allows the pharmaceutical composition of the present invention to release 80% or more of the active ingredient within 6 hours, thereby exhibiting the drug efficacy through only once-daily administration. The administration of less than the above content is insufficient to exhibit the above effect, and the administration of more than the above content is not economical, and increases the entire weight of the unit dosage form, causing an inconvenience of administration.

According to still another aspect of the present invention, the composition of the present invention may further contain a coating agent. An example of the coating agent may be at least one selected from the group consisting of hydroxypropyl methylcellulose, polyvinyl alcohol, polypropylene glycol, acrylic acid polymers, polymethacrylate copolymers, polyethylene glycol, polyvinyl acetate, and a mixture thereof, but is not limited thereto.

In addition, the composition may further contain a pharmaceutically acceptable excipient, and a general excipient that can improve the manufacturing, external appearance, and compression of a drug may be used without deteriorating biological activity and characteristics of the drug. The specific content thereof may be determined by the solubility and chemical characteristics of the active ingredient, selected route of administration, and standard pharmaceutical practice. Examples of the pharmaceutically acceptable excipient may include a diluent, a binder, a lubricant, a preservative, a stabilizer, an anti-adhesive, a fluidizer, or a colorant.

More specifically, starch, microcrystalline cellulose, lactose, glucose, mannitol, alginate, dicalcium phosphate, etc., may be used as the diluent; hydroxypropyl cellulose, polyethylene glycol, polyvinyl pyrrolidone, polyvinyl alcohol, etc., may be used as the binder; and light anhydrous silicic acid, talc, magnesium stearate, calcium stearate, zinc stearate, sodium stearyl fumarate, lauryl sodium sulfate, hydrogenated vegetable oil, sodium benzoate, glyceryl monostearate, glyceryl palmitostearate, glyceryl behenate, sodium stearyl fumarate, polyethylene glycol, etc., may be used as the slip modifier, but are not limited thereto. Besides, various additives, such as an antioxidant, a colorant, a flavoring agent, a preservative, and a taste blocker, may be selected and used within general dose ranges by a person skilled in the art.

According to an embodiment of the present invention, the pharmaceutical composition of the present invention has an acebrophylline dissolution rate of 20-50 wt% (more specifically, 30-50 wt%, and still more specifically, 35-50 wt%) at 1 hour after dissolution, and an acebrophylline dissolution rate of 75-100 wt% (more specifically, 80-100 wt%, and still more specifically, 80-95 wt%) at 6 hours after dissolution. Thus, when administered into the body, the pharmaceutical composition may exhibit favorable drug efficacy even under a reduced frequency of administration, for example, once daily. Therefore, the patient convenience of administration can be improved by using the pharmaceutical composition.

As used herein, the term "dissolution rate", unless otherwise mentioned, is obtained by: initiating a dissolution test in 500 mL of a dissolution medium (first fluid in Korean pharmacopoeia disintegration test) of pH 1.2 under 37±0.5□ and 50 rpm using method 2 (paddle method) of Korean pharmacopoeia; carefully adding 400 mL of a test solution 2(second fluid, 0.235 M anhydrous Na₂HPO₄ solution) to the dissolution medium of pH 1.2 to make a liquid of pH 6.8 at 2 hours after the initiation of the dissolution test; and then continuously performing the dissolution test. The first fluid is prepared by dissolving 2.0 g of sodium chloride in 7.0 mL of hydrochloric acid and water to make 100 mL, and the second fluid is prepared by adding 118 mL of 0.2 mol/L sodium hydroxide solution and water to 250 mL of 0.2 mol/L potassium dihydrogen phosphate solution to make 100 mL.

The pharmaceutical composition of the present invention may preferably be formulated in an administration form suitable to be orally administered, but is not limited thereto, and may preferably be provided in a solid oral dosage form in consideration of the productivity of producers and the convenience of administration and portability of patients.

As a specific example, the solid oral dosage form may be, for example, a single tablet, a multi-layered tablet, such as a double-layered tablet or a triple-layered tablet, a core tablet, a coated tablet, or a capsule, but is not limited thereto. As a preferable example, the solid oral dosage form may be a monolithic matrix type sustained-release tablet containing acebrophylline or a salt thereof as an active ingredient, a hydrophilic sustained-release agent for controlling the dissolution of the active ingredient, an excipient, and a coating agent.

According to an embodiment of the present invention, the composition of the present invention is provided in the form of a monolithic matrix type tablet.

According to an embodiment of the present invention, the composition of the present invention has both immediate-release characteristics and sustained-release characteristics. As used herein to recite the composition of the present invention, unless otherwise mentioned, the term "immediate-release characteristics" refers to the rapid release of a drug at an initial stage after administration (e.g., at 1-4 hours, 1-3 hours, 1.5-4 hours, 1.5-3 hours, or 1,5-2,5 hours after administration). The composition of the present invention has both immediate-release characteristics in which a drug can rapidly reach the effective blood concentration after administration (e.g., oral administration) to exhibit drug efficacy, and sustained-release characteristics in which a drug efficacy can be retained for a long period of time.

According to an embodiment of the present invention, the composition of the present invention shows the maximal blood ambroxol concentration at 1-4 hours (more specifically, 1-3 hours, 1.5-4 hours, 1.5-3 hours, or 1.5-2.5 hours) after once daily oral administration, thus exhibiting immediate-release characteristics, and then exhibits sustained-release characteristics.

The particular type or dose of the agent of the present invention may be selected depending on characteristics of the patient, especially, age, body weight, and lifestyle of the patient and, in some cases, by a doctor, and preferably, the composition of the present invention may be prepared such that 200 mg of acebrophylline is administered once daily in order to improve patient compliance.

Each dosage form usable in the present invention may be obtained by methods generally known in the art. For example, dry granulation, wet granulation, melt granulation, fluidized-bed granulation, direct compression, molding, compressive molding, and the like may be carried out. Preferably, dry granulation, wet granulation, and melt granulation may be carried out. For example, in the case of direct compression method, the composition of the present invention may be prepared in an oral dosage form by mixing acebrophylline and a pharmaceutically acceptable excipient, followed by tableting and coating. In addition, in the case of dry granulation method, the composition of the present invention may be prepared by mixing acebrophylline and a pharmaceutically acceptable excipient, and then forming granules from the mixture using a compression granulator(roller compactor), followed by sieving, tableting, and coating. In the case of wet granulation method, the composition of the present invention may be prepared by mixing acebrophylline and a pharmaceutically acceptable excipient, forming wet granules from the mixture using a liquid binder, and then drying the wet granules, followed by sieving, tableting, and coating.

The acebrophylline-containing preparation, according to the present invention, is very useful since the preparation is orally administered to exhibit a prompt therapeutic effect; the active ingredient is continuously sustained at the predetermined concentration in the blood plasma for a considerable time; the dosing frequency is decreased to once daily; and resultantly, with respect to patients, the drug adaptability is increased and the drug compliance is improved.

In accordance with another aspect of the present invention, there is provided a method for imparting initial immediate-release characteristics and late sustained-release characteristics to a pharmaceutical composition containing acebrophylline, the method including a step of adding, to the pharmaceutical composition, a hydrophilic sustained-release agent having a viscosity of 13,000 cps or less, followed by mixing.

Since the method of the present invention is directly associated with the above-described pharmaceutical composition, descriptions of overlapping contents between the two are omitted to avoid excessive complexity of the specification due to repetitive descriptions thereof.

As used herein, the term "initial immediate-release" refers to an acebrophylline dissolution rate of 20-50 wt% at 1 hour in the dissolution liquid of pH 1.2 when the dissolution rate is analyzed by the above-described paddle method, or a rapid release (e.g., exhibiting the maximal blood concentration) of a drug (for example, at 1-4 hours, 1-3 hours, 1.5-4 hours, 1.5-3 hours, or 1.5-2.5 hours after administration) when a pharmaceutical composition (e.g., tablet) is administered to the human body (e.g., oral administration).

As used herein, the term "late sustained-release" refers to a sustained-release occurring after the dissolution of acebrophylline occurs by the foregoing initial immediate-release. For example, the pharmaceutical composition of the present invention may exhibit an initial immediate-release, a rapidly reduced release rate, a reduced release rate, and then sustained-release. Alternatively, the pharmaceutical composition of the present invention may exhibit an initial immediate-release, a gently reduced release rate, and then sustained-release at such a reduced release rate.

The method of the present invention may be performed in various manners.

For example, for manufacturing tablets, the present invention may be performed by including: (a) mixing a diluent and a binder with acebrophylline as an active ingredient (optionally, a lubricant and a disintegrant may be added), followed by granulation; and (b) adding a hydrophilic sustained-release agent having a viscosity of 13,000 cps or less, capable of imparting initial immediate-release characteristics and late sustained-release characteristic to the resultant material in step (a) (optionally, a lubricant may be added), followed by mixing and tableting. Optionally, the tablet prepared after the tableting may be coated.

According to an embodiment of the present invention, the pharmaceutical composition, which is mixed with the hydrophilic sustained-release agent having a viscosity of 13,000 cps or less, has an acebrophylline dissolution rate of 20-50 wt% (more specifically, 30-50 wt%, and still more specifically, 35-50 wt%) at 1 hour after dissolution, and an acebrophylline dissolution rate of 75-100 wt% (more specifically, 80-100 wt%, and still more specifically, 80-95 wt%) at 6 hours after dissolution.

According to an embodiment of the present invention, the hydrophilic sustained-release agent used herein is at least one selected from the group consisting of agar, carbomer, alginic acid, sodium alginate, carrageenan, guar gum, acacia, hypromellose (HPMC), hypromellose acetate succinate, hypromellose phthalate, methylcellulose, polyethylene oxide, polyvinyl alcohol, sodium hyaluronate, xanthan gum, polycarbophil, povidone, ceratonia, chitosan, and a mixture thereof.

According to an embodiment of the present invention, the pharmaceutical composition mixed with the hydrophilic sustained-release agent is provided in a solid oral dosage form.

According to an embodiment of the present invention, the pharmaceutical composition mixed with the hydrophilic sustained-release agent is provided in the form of a monolithic matrix type tablet.

According to an embodiment of the present invention, the pharmaceutical composition mixed with the hydrophilic sustained-release agent shows a maximal blood ambroxol concentration at 1-4 hours after once daily oral administration, thereby exhibiting immediate-release characteristics, and then exhibits sustained-release characteristics.

### Advantageous Effects

The acebrophylline-containing monolithic matrix type sustained-release preparation provided according to the present invention is designed to allow a simple preparation process and to exhibit both a fast-acting property and a long-acting property, and thus can secure an excellent therapeutic effect with only once daily administration and can increase drug adaptability, thereby expecting a significantly improved compliance of administration and excellent therapeutic effect.

### Brief Description of the Drawings

FIG. 1 shows dissolution test results of preparations obtained according to example 1-1 and comparative example 1.
FIG. 2 shows the results of in *vivo* pharmacokinetics (PK) test in beagle dog of example 1-1.

### Mode for Carrying Out the Invention

Hereinafter, the present invention will be described in detail with reference to the following examples. However, the following examples are merely for illustrating the present invention and are not intended to limit the scope of the present invention.

### Example 1: Manufacture of hydrophilic sustained-release preparation containing HPMC having viscosity of 10,000 cps or less

Components 1 to 5 on table 1 were mixed, and made into granules. Components 6 to 8 on table 1 were added to the granules, followed by mixing and tableting. After the tableting, the tablets were coated with component 9 as a coating agent on table 1.

METHOCEL K100LV, METHOCEL E4M, and METHOCEL E10MCR, which were used to manufacture example 1, were HPMCs having 100, 4,000, and 10,000 cps, respectively.

**[Table 1]**

| | Ingredient | Weight ratio (%) | |
|---|---|---|---|
| | | Example 1-1 | Example 1-2 |
| 1 | Acebrophylline (active pharmaceuticalingredient) | 45.6 | 45.6 |
| 2 | Microcrystalline cellulose(diluent) | 34.2 | 37.4 |
| 3 | Talc(lubricant) | 2.3 | 2.3 |
| 4 | Croscarmellose sodium(disintegrant) | 5.7 | 5.7 |
| 5 | Hypromellose (METHOCEL K100LV) (binder) | 4.6 | 4.6 |
| 6 | Hypromellose(METHOCEL E4M) (sustained-release agent) | 6.8 | - |
| 7 | Hypromellose (METHOCEL E10MCR) (sustained-release agent) | - | 3.5 |
| 8 | Magnesium stearate(lubricant) | 0.9 | 0.9 |
| | Total (plain tablet) | 100.0 | 100.0 |
| 9 | Opadry (coating agent) | 3.0 | 3.0 |
| | Total (coated tablet) | 103.0 | 103.0 |

### Example 2: Manufacture of hydrophilic sustained-release preparation containing polyethylene oxide

Components 1 to 5 on table 2 were mixed, and made into granules. Components 6 to 7 on table 2 were added to the granules, followed by mixing and tableting. After the tableting, the tablets were coated with component 8 as a coating agent on table 2.

The viscosity of polyethylene oxide (Polyox WSR N-60K), which is used to manufacture example 2, was 2,000-4,000 cps.

**[Table 2]**

| | Ingredient | Weight ratio (%) |
|---|---|---|
| | | Example 2 |
| 1 | Acebrophylline | 45.6 |
| 2 | Microcrystalline cellulose | 29.6 |
| 3 | Talc | 2.0 |
| 4 | Croscarmellose sodium | 5.7 |
| 5 | Hypromellose (METHOCEL K100LV) | 4.2 |
| 6 | Polyethylene oxide (Polyox WSR N-60K) | 12.0 |
| 7 | Magnesium stearate | 0.9 |
| | Total (plain tablet) | 100.0 |
| 8 | Opadry | 3.0 |
| | Total (coated tablet) | 103.0 |

### Example 3: Manufacture of hydrophilic sustained-release preparation containing carbomer

Components 3 to 5 on table 1 were mixed, and made into granules. Components 6 to 7 on table 3 were added to the granules, followed by mixing and tableting. After the tableting, the tablets were coated with component 8 as a coating agent on table 3.

The viscosity of Carbopol 71G used to manufacture example 3 was 4,000-11,000 cps.

**[Table 3]**

| | Ingredient | Weight ratio (%) |
|---|---|---|
| | | Example 3 |
| 1 | Acebrophylline | 45.6 |
| 2 | Microcrystalline cellulose | 35.1 |
| 3 | Talc | 2.0 |
| 4 | Croscarmellose sodium | 5.7 |
| 5 | Hypromellose (METHOCEL K100LV) | 4.2 |
| 6 | Carbomer (CARBOPOL 71G) | 6.5 |
| 7 | Magnesium stearate | 0.9 |
| | Total (plain tablet) | 100.0 |
| 8 | Opadry | 3.0 |
| | Total (coated tablet) | 103.0 |

### Example_4: Manufacture of hydrophilic sustained-release preparation containing xanthan gum and alginate

Components 1 to 5 on table 4 were mixed, and made into granules. Components 6 to 8 on table 4 were added to the granules, followed by mixing and tableting. After the tableting, the tablets were coated with component 9 as a coating agent on table 4.

The viscosities of xanthan gum and alginate used to manufacture example 4 were 1,200-1,600 cps.

**[Table 4]**

| | Ingredient | Weight ratio (%) | Weight ratio (%) |
|---|---|---|---|
| | | Example 4-1 | Example 4-2 |
| 1 | Acebrophylline | 45.6 | 45.6 |
| 2 | Microcrystalline cellulose | 29.4 | 23.4 |
| 3 | Talc | 2.2 | 2.2 |
| 4 | Croscarmellose sodium | 5.7 | 5.7 |
| 5 | Hypromellose (METHOCEL K100LV) | 4.2 | 4.2 |
| 6 | Xanthan gum | 6.0 | 6.0 |
| 7 | Alginic acid | 6.0 | 12.0 |
| 8 | Magnesium stearate | 0.9 | 0.9 |
| | Total (plain tablet) | 100.0 | 100.0 |
| 9 | Opadry | 3.0 | 3.0 |
| | Total(coated tablet) | 103.0 | 103.0 |

### Comparative example 1: Manufacture of hydrophilic sustained-release preparation containing HPMC having viscosity of more than 13,000 cps

The preparation of comparative example 1 was formed using components 1 to 9 on table 5. HPMC K100M and HPMC K15M, which were used as a hydrophilic sustained-release agent in comparative example 1, had viscosities of 100,000 cps and 15,000 cps, respectively.

**[Table 5]**

| | Ingredient | Comparative example 1 | Note |
|---|---|---|---|
| | | Weight ratio (%) | |
| 1 | Acebrophylline | 66.7 | Sustained-release layer |
| 2 | Microcrystalline cellulose | 6.7 | |
| 3 | Calcium phosphate dihydrate | 8.3 | |
| 4 | Carboxymethyl cellulose sodium | 1.7 | |
| 5 | Hypromellose (HPMC K100M) | 8.3 | |
| 6 | Hypromellose (HPMC K15M) | 1.7 | |
| 7 | Povidon K 30 | 5.0 | |
| 8 | Talc | 0.7 | |
| 9 | Magnesium stearate | 1.0 | |
| | Total (plain tablet) | 100.0 | |

### Test Example 1: Comparative test of dissolution rate between present invention and comparative example

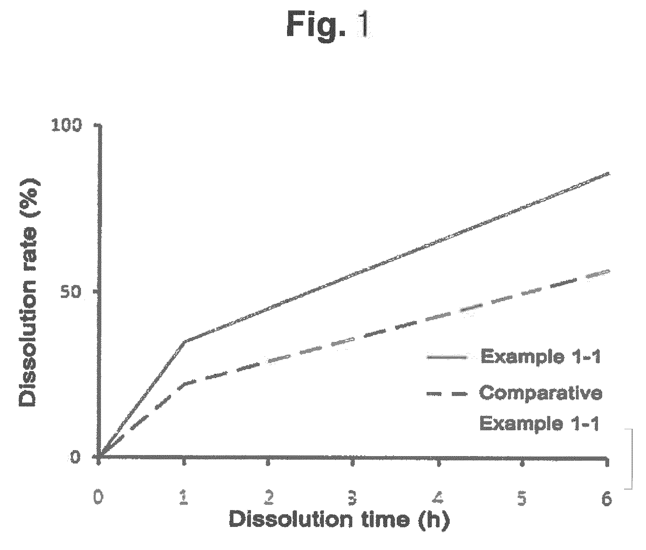

With respect to preparations of example 1-1 and comparative example 1, a dissolution test was initiated in 500 mL of a dissolution medium(first fluidin Korean pharmacopoeia disintegrationtest) of pH 1.2 under 37±0.5□ and 50 rpm using the method2 (paddle method) of Korean pharmacopoeia. At 2 hours after the initiation of the dissolution test, 400 mL of a test solution 2(second fluid, 0.235 M anhydrous Na₂HPO₄ solution) was added to the dissolution liquid of pH 1.2 to make a liquid of pH 6.8, and then the dissolution test was continuously carried out. The dissolution rate was analyzed by taking the dissolution liquid at the prescribed dissolution time and checking the concentration of acebrophylline through HPLC. The dissolution rate test results over the time are shown in FIG. 1.

HPLC analysis conditions
- mobile phase:acetonitrile :0.1 % ammonium carbonate solution = 570 : 430
- column:C18, 5µm 4.0 mm x 125 mm or similar column
- flow rate:1.0 mℓ/min
- analysis wavelength:UV detector (244 nm)

As a result of the test, as shown in FIG. 1, the initial dissolution rate of example 1-1 using the sustained-release agent having a viscosity of 13,000 cps or less was excellent compared with that of comparative example 1 using the sustained-release agent having a viscosity of more than 13,000 cps, and with the passage of time, the preparation of example 1-1 showed more excellent dissolution than comparative example 1. More specifically, the initial dissolution rate (dissolution rate at 1 hour after dissolution) of the preparation of comparative example 1 was about 22%, whereas the preparation of example 1-1 showed an initial dissolution rate of about 37%, corresponding to an excellent initial dissolution rate. In addition, with the passage of 1 hour after dissolution, the preparation of example 1-1 exhibited sustained-release characteristics. The dissolution rate at 6 hours after dissolution was about 52% for comparative example 1, but about 83% for example 1-1, and thus the preparation of the presentinvention exhibited excellent dissolution characteristics even after the initial dissolution.

As such, the use of the sustained-release agent having a viscosity of 13,000 cps or less led to an excellent initial dissolution rate, thus exhibiting fast drug efficacy, and even with the passage of time, showed an excellent dissolution rate compared with comparative example 1, thereby securing a fast effect and a continuous effect through only once daily administration, and thus can improve the convenience of administration and expect the improved therapeutic effect.

### Test Example 2: In vivo beagle dog PK

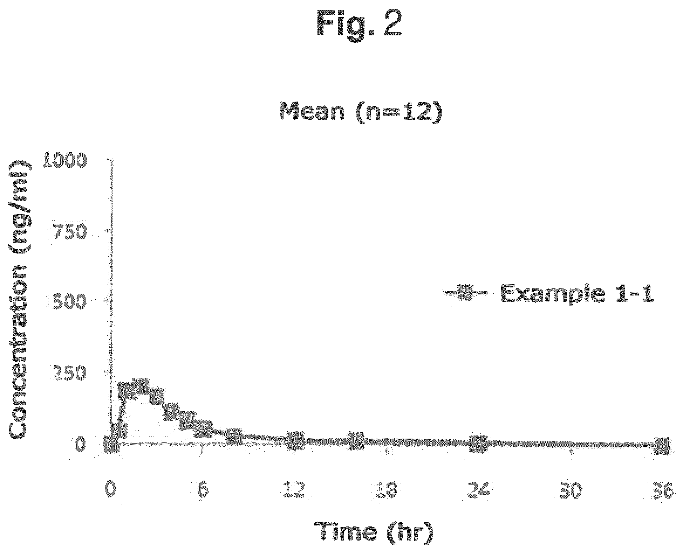

The blood ambroxol concentration was measured when the preparation (200 mg) of example 1-1 (HPMC having a viscosity of 13,000 cps or less) was administered once daily to twelve beagle dogs (male, 9-12 kg, aged 20-30 months) with an empty stomach (table 7 and FIG. 2).

As the pretreatment procedure for measuring the blood ambroxol concentration, 50 mL of a plasma sample was placed in a micro-tube, and 10 mL of a sildenafil citrate solution (10 ug/mL in 50% methanol), as an internal standard material, and 100 mL of 0.05 M a NaOH solution were added thereto, followed by vortexing for 5 seconds. 2 mL of an extraction solvent (methyl tert-butyl ether) was added thereto, followed by vortexing for 2 minutes and centrifugation at 4,000 rpm for 5 minutes. The supernatant was taken, and completely concentrated and dried in a nitrogen evaporator, and then again dissolved in 300 mL of a mixture of 0.1% formic acid and 50% methanol at 1:1 (v/v), and of this mixture, 5 mL was taken and injected into LC-MS/MS.

### <LC-MS/MS analysis conditions>

-
Detector:MS/MS(ESI negative ion, MRM mode)
Ambroxol:m/z 378.99 > 263.91
Sildenafil citrate (internal standard material):m/z 475.22 > 100.19
Desolvation temp.: 500□
Desolvation gas flow:600 L/hr
Capillary voltage:1000 V
Collision gas:Argon
Nebulizing gas:Nitrogen

- Column:Fortis C18 (2.1 X 5.0 mm, 1.7 mm particle size, Fortis)
- Data processor:Target Lynx 4.1
- Mobile phase:0.1% formic acid :methanol = 30 :70 (v/v)
- Flow rate:0.2 mL/min
- Injection dose:5 mL

**[Table 7]**

| PK parameters | Example 1-1 | T/R |
|---|---|---|
| | Mean ± SD | |
| AUCₜ (ng·hr /mL) | 1304.17 ± 794.78 | 0.80 |

As a result, the pharmaceutical composition containing a hydrophilic sustained-release agent having a viscosity of 13,000 cps or less, of the present invention, showed a considerable blood ambroxol concentration through only once daily administration. In addition, the preparation of example 1-1 reached a blood concentration of about 210 ng/mL at about 1.8 hours after administration to exhibit initial immediate-release characteristics, and then exhibits sustained-release characteristics.

## Claims

1. A sustained-release pharmaceutical composition containing acebrophylline and a hydrophilic sustained-release agent having a viscosity of 13,000 cps or less.

2. The composition of claim 1, wherein the hydrophilic sustained-release agent is contained in less than 20 wt% on the basis of the entire composition.

3. The composition of claim 1, wherein the composition has an acebrophylline dissolution rate of 20-50 wt% at 1 hour after dissolution and an acebrophylline dissolution rate of 75-100 wt% at 6 hours after dissolution.

4. The composition of claim 1, wherein the composition is administered once daily.

5. The composition of claim 1, wherein the hydrophilic sustained-release agent is at least one selected from the group consisting of agar, carbomer, alginic acid, sodium alginate, carrageenan, guar gum, acacia, hypromellose (HPMC), hypromellose acetate succinate, hypromellose phthalate, methylcellulose, polyethylene oxide, polyvinyl alcohol, sodium hyaluronate, xanthan gum, polycarbophil, povidone, ceratonia, chitosan, and a mixture thereof.

6. The composition of claim 1, wherein the composition is provided in a solid oral dosage form.

7. The composition of claim 1, wherein the composition is used for the prevention or treatment of at least one selected from the group consisting of acute respiratory disease, chronic respiratory disease, acute bronchitis, chronic bronchitis, bronchial asthma, sinusitis, and rhinitis sicca.

8. The composition of claim 1, wherein the composition has both immediate-release characteristics and sustained-release characteristics.

9. The composition of claim 8, wherein the composition shows the maximal blood ambroxol concentration at 1-4 hours after once daily oral administration to exhibit immediate-release characteristics, and then exhibits sustained-release characteristics.

10. A method for imparting initial immediate-release characteristics and late sustained-release characteristics to a pharmaceutical composition containing acebrophylline, the method comprising a step of adding, to the pharmaceutical composition, a hydrophilic sustained-release agent having a viscosity of 13,000 cps or less, followed by mixing.

11. The method of claim 10,wherein the pharmaceutical composition mixed with the hydrophilic sustained-release agent has an acebrophylline dissolution rate of 20-50 wt% at 1 hour after dissolution and an acebrophylline dissolution rate of 75-100 wt% at 6 hours after dissolution.

12. The method of claim 10,wherein the hydrophilic sustained-release agent is at least one selected from the group consisting of agar, carbomer, alginic acid, sodium alginate, carrageenan, guar gum, acacia, hypromellose (HPMC), hypromellose acetate succinate, hypromellosephthalate, methylcellulose, polyethylene oxide, polyvinyl alcohol, sodium hyaluronate, xanthan gum, polycarbophil, povidone, ceratonia, chitosan, and a mixture thereof.

13. The method of claim 10,wherein the pharmaceutical composition mixed with the hydrophilic sustained-release agent is provided in a solid oral dosage form.

14. The method of claim 10,wherein the pharmaceutical composition mixed with the hydrophilic sustained-release agent shows the maximal blood ambroxolconcentration at 1-4 hours after once daily oral administration to exhibit immediate-release characteristics, and then exhibits sustained-release characteristics.
